# EUROPÄISCHE PATENTSCHRIFT

(11) **EP 1 948 263 B1**
(45) Veröffentlichungstag und Bekanntmachung des Hinweises auf die Patenterteilung: **03.07.2013**
(21) Anmeldenummer: 06818569.3
(22) Anmeldetag: 16.11.2006
(51) Int. Cl.: A61B 17/11, A61L 27/22, A61L 27/50, A61L 27/56

(54) **NERVENLEITSCHIENE**
NERVE GUIDE
TUYAUX DE GUIDAGE POUR LES NERFS

(30) Priorität: 17.11.2005 DE 102005054941
(43) Veröffentlichungstag der Anmeldung: 30.07.2008
(73) Patentinhaber: GELITA AG, 69412 Eberbach (DE)
(72) Erfinder: AHLERS, Michael, 69412 Eberbach (DE); SCHLOSSHAUER, Burkhard, 72074 Tübingen (DE); DREESMANN, Lars, 88433 Schemmerhofen (DE); LIETZ, Martin, 72127 Kusterdingen-Immenhausen (DE)
(74) Vertreter: Hoeger, Stellrecht & Partner Patentanwälte
(86) Internationale Anmeldenummer: PCT/EP2006/010976
(87) Internationale Veröffentlichungsnummer: WO 2007/057177

(56) Entgegenhaltungen:
- EP-A- 1 254 671
- EP-A1- 0 945 145
- EP-A2- 1 201 256
- WO-A-03/086290
- WO-A-2004/071736
- WO-A-2005/111121
- CHEN YUEH-SHENG ET AL: "An in vivo evaluation of a biodegradable genipin-cross-linked gelatin peripheral nerve guide conduit material." BIOMATERIALS JUN 2005, Bd. 26, Nr. 18, Juni 2005 (2005-06), Seiten 3911-3918, XP004697270 ISSN: 0142-9612

## Beschreibung

Die Erfindung betrifft eine Nervenleitschiene, umfassend einen Formkörper aus einem resorbierbaren Material.

Nervenleitschienen der eingangs beschriebenen Art werden bei Läsionen des Nervensystems verwendet um an die Enden von verletzten Nerven anzuknüpfen und die Lücke zwischen den beiden Enden zu überbrücken. Dabei bietet die Nervenleitschiene den Nervenfasern (Axone) einen Raum um zu wachsen und bietet idealerweise andererseits Schutz vor dem Eindringen von Narben bildenden Bindegewebszellen (Fibroblasten).

Dabei nehmen die beiden Enden der Nervenleitschiene die beiden bei der Läsion entstandenen Nervenstümpfe auf und überbrücken die dazwischen bestehende Lücke. Das zwischen den Nervenstümpfen verbleibende Lumen der Nervenleitschiene gibt den sich regenerierenden Axonen die Richtung vor und vermeidet ein fehlgeleitetes Wachstum derselben, wodurch eine zielgerichtete Regeneration gefördert wird.

Biologisch abbaubare oder resorbierbare Nervenleitschienen sind dabei gegenüber nicht-resorbierbaren bevorzugt, da nach einer Wiederherstellung der Nervenfasern oder in deren Verlauf die Nervenleitschiene abgebaut wird und damit im Gegensatz zu den nicht-resorbierbaren Nervenleitschienen eine u.U. notwendige weitere Operation zur Entfernung derselben vermieden wird, die selbst wiederum ein Risiko der Beschädigung der Nervenfasern mit sich bringt. Allerdings muss bei der resorbierbaren Nervenleitschiene die Stabilität gegen Resorptionsmechanismen einstellbar sein, was eine Herausforderung darstellt.

Ziel der Nervenregeneration mittels Nervenleitschienen ist die Wiederherstellung motorischer und sensorischer Funktionen sowie das Unterbinden von fehlgeleitetem Nervenwachstum und schmerzhafter Neurombildung.

Die regenerative Medizin kann für Verletzungen der Nervenfasern des Nervensystems bisher nur unzureichende Therapien anbieten. Obwohl die meisten adulten Neuronen im Prinzip die Fähigkeit behalten, Axone zu regenerieren, findet ohne Hilfestellung im peripheren Nervensystem nur eine begrenzte funktionelle Regeneration und im zentralen Nervensystem so gut wie gar keine statt.

Gründe für diese Beschränkungen sind unter anderem der Kontaktverlust mit der ursprünglichen Nervenbahn und inhibitorische Narbenbildungen.

Eine operative Überbrückung läsionierter/inhibitorischer Areale stellt im Prinzip eine erfolgreiche Therapiestrategie dar, für die in der Praxis allerdings bisher fast ausschließlich autologe Nerventransplantate (meist Suralnerv des Unterschenkels) verwendet werden.

Die damit verbundenen Nachteile wie Morbidität im Donorbereich und limitierte Verfügbarkeit haben die Entwicklung von synthetischen Nervenleitfasern wesentlich stimuliert. Nervenleitfasern als Hohlröhren wurden in jüngerer Zeit aus verschiedenen inerten und resorbierbaren rein synthetischen Polymeren und biologischen Komponenten wie z.B. Polysacchariden, Kollagen oder spezifisch vernetzten Gelatinematerialien entwickelt.

In der WO 2005/111121 A2 (nachveröffentlicht) werden Nervenleitschienen offenbart, die einen Formkörper auf Basis von vernetzter Gelatine umfassen.

Chen et al. (Biomaterials 26 (2005) 3911-3918) beschreiben resorbierbare Nervenleitschienen auf Basis von Gelatine, die mit Genipin vernetzt ist.

Die EP 1 254 671 A1 offenbart ein Instrument zur Regenerierung eines Gewebes oder Organs, insbesondere von Nerven, umfassend einen Träger und eine schwammartige feine Matrix im Inneren des Trägers, die beide aus einem biologisch abbaubaren oder bioabsorbierbaren Material gebildet sind, und einen linearen Kanal zur Führung eines Gewebes oder Organs eines lebenden Organismus, wobei der lineare Kanal wenigstens eine Faser im Inneren des Trägers in Längsrichtung desselben umfasst.

Die EP 0 945 145 A1 offenbart einen künstlichen Schlauch für Nerven, der einen Schlauch mit Beschichtungen aus Gelatine oder Kollagen auf der Innen- und Außenoberfläche des Schlauchs umfasst, sowie einen Kollagenkörper mit Hohlräumen in seinem Lumen, die den Schlauch im Wesentlichen parallel zu seiner Achse durchlaufen, wobei die Hohlräume mit einem Matrixgel gefüllt sind, das Kollagen, Laminin, Heparansulfat-Proteoglykane, Entactin und Wachstumsfaktor enthält.

Aufgabe der vorliegenden Erfindung ist es, die eingangs beschriebene Nervenleitschiene weiter zu verbessern, um eine möglichst ungehinderte Entwicklung der Axone bei der Regeneration zu erlauben.

Diese Aufgabe wird erfindungsgemäß dadurch gelöst, dass die eingangs genannte Nervenleitschiene einen Formkörper aus einem vernetzten, resorbierbaren, Gelatine basierenden Material umfasst, wobei der Formkörper ein röhrchenförmlger Hohlkörper ist mit einer Wandung und einer Außenoberfläche und einer Innenoberfläche, die ein Lumen definiert, wobei die Nervenleitschiene eine das Lumen umgebende, semipermeable Schicht umfasst, und wobei
(i) der Vernetzungsgrad der Nervenleitschiene bzw. deren Teile, insbesondere deren Formkörper, an einem Ende der Nervenleitschiene höher ist als am anderen Ende und in Richtung zu diesem anderen Ende hin in mehreren Stufen oder im wesentlichen kontinuierlich abnimmt; oder
(ii) der Vernetzungsgrad der Nervenleitschiene bzw. deren Teile, insbesondere deren Formkörper, an deren Enden geringer ist als im Bereich zwischen den Enden.

Aus Gelatine basierenden Materialien lassen sich gut sterilisierbare Nervenleitschienen als Implantate bilden, die auch über längere Zeit, insbesondere auch bei Raumtemperatur, lagerfähig sind. Auch lassen sich solche Nervenleitschienen noch im Operationssaal in der Länge und an sonstige Belange anpassen.

Gelatine basierende Materialien lassen sich auch definiert und in der Zusammensetzung sowie den Resorptionseigenschaften reproduzierbar herstellen. Außerdem erweisen sich solche Materialien bezüglich ihrer (patho)physiologischen Reaktionen kalkulierbar. Ferner weisen die Materialien generell die benötigte Biokompatibilität auf und sind weder toxisch, infektiös noch entzündungsfördernd.

Darüber hinaus sind die Gelatine basierenden Materialien auch geeignet um Nervenleitschienen herzustellen, die den mechanischen Anforderungen eines Implantats genügen. Gleichzeitig lassen sich die Materialien so formulieren, dass sie eine ausreichende Flexibilität während der Handhabung durch den Chirurgen bei der Operation als auch postoperativ als eingesetztes Implantat aufweisen, so dass eine Kompression der Nerven vermieden und eine Anpassung bei Bewegungen von Körperteilen des behandelten Patienten möglich wird.

Andererseits kann mit den erfindungsgemäß für den Formkörper verwendeten Gelatine basierenden Materialien eine ausreichende Formstabilität, die ein Kollabieren des eingesetzten Implantats vermeidet, sichergestellt werden.

Weiter lassen sich ausreichend reißfeste Nervenleitschienen herstellen, die ein Annähen des Implantats an die bei der Läsion entstandenen Nervenstümpfe erlauben.

Gemäß der vorliegenden Erfindung ist eine Schicht der Nervenleitschiene, die Teil des Formkörpers sein kann, semi-permeabel ausgebildet. Dies bedeutet, dass eine Nährstoff- und Gasdiffusion zwischen Lumen und der Umgebung der Nervenleitschiene in radialer Richtung möglichst ungehindert möglich ist, während andererseits eine Diffusion unerwünschter Substanzen, insbesondere auch das Eindringen von Zellen, wie z.B. Fibroblasten, aus dem Umgebungsgewebe unterbunden wird.

Zur Realisierung dieser Schutzfunktion der semi-permeablen Schicht bieten sich verschiedene Möglichkeiten, die teilweise untereinander auch kombiniert werden können.

So kann die semi-permeable Schicht der Nervenleitschiene z.B. Poren aufweisen, die im Mittel kleiner als ca. 0,5 µm sind. Durch solche so genannten Nanoporen können Zellen nicht passieren, während andererseits eine Nährstoff- und Gasdiffusion durch solche Poren hindurch nahezu ungehindert geschehen kann.

Alternativ hierzu lässt sich eine Gelstruktur verwenden, die einerseits die Nährstoff- und Gasdiffusion nach wie vor erlaubt, andererseits als Zellbarriere wirkt.

Bei einer anderen Ausführungsform weist die Nervenleitschiene als semipermeable Schicht eine Sperrschicht auf, welche für positiv geladene Spezies, insbesondere auch Zellen, insbesondere Fibroblasten, im Wesentlichen undurchlässig ist, da Zellen häufig an ihrer Oberfläche positive Ladungen tragen und deshalb auf positiv geladenen Oberflächen schlecht adhärieren.

Eine andere Ausführungsform weist eine semipermeable Schicht auf, die eine extrem hohe Hydrophilie aufweist und auch hier wird beobachtet, dass eine Zelldiffusion durch eine solche Schicht stark behindert wird.

Ein ähnlicher Effekt lässt sich auch mit einer Schicht erzielen, welche hydrophob ist. Auch hier ist die Zellmigration deutlich reduziert.

Hydrophobe Schichten können beispielsweise aus einem Material gebildet werden, welches eine mit Fettsäureresten modifizierte Gelatine umfasst. Ein Beispiel hierfür ist die Dodecenyl-succinierte Gelatine.

Bevorzugt wird bei solchen Materialien die Gelatine an den Aminogruppen der Lysingruppen, insbesondere an 10 bis 80 % der Lysingruppen, mit Fettsäureresten modifiziert sein.

Schließlich steht als weitere Variante zur Verfügung, dass die Nervenleitschiene oder deren Formkörper an der Außenoberfläche immobilisierte Repulsionproteine als semipermeable Schicht aufweisen, z.B. Semaphorine, die eine Zelleinwanderung behindern.

Die weiter oben schon angesprochene mechanische Festigkeit der erfindungsgemäßen Nervenleitschienen muss bei der Applikation, d.h. bei der Verwendung als Implantat, ein Einnähen erlauben. Die dafür benötigte Festigkeit, insbesondere auch Ausreißfestigkeit gegenüber den verwendeten Nähmaterialien, lässt sich insbesondere durch die Verwendung von Verstärkungsstoffen, die insbesondere in das Gelatine basierende Material eingebettet sind, erzielen. Die Verstärkungsstoffe sollen physiologisch verträglich und am besten ebenfalls resorbierbar sein.

Je nach Art des Verstärkungsstoffs lässt sich neben der Beeinflussung der mechanischen Eigenschaften auch die Stabilität gegen Resorptionsmechanismen in gewissem Umfang beeinflussen. Insbesondere lässt sich die Resorptionsstabilität der Verstärkungsstoffe unabhängig von den anderen Komponenten, z.B. dem Gelatine basierenden Material, der Nervenleitschiene wählen.

Die Verstärkungsstoffe zeigen schon bei Anteilen von 5 Gew.% (bezogen auf die Trockenmasse) eine merkliche Verbesserung der mechanischen Eigenschaften der Nervenleitschienen.

Oberhalb von Anteilen von 60 Gew.% lässt sich in der Regel keine signifikante Verbesserung mehr erreichen und/oder die gewünschten Resorptionseigenschaften oder auch die notwendige Flexibilität der Nervenleitschienen lassen sich nur noch schwierig darstellen.

Die Verstärkungsstoffe lassen sich aus partikulären und molekularen Verstärkungsstoffen sowie Mischungen hiervon auswählen.

Bei den partikulären Verstärkungsstoffen empfiehlt sich insbesondere die Verwendung von Verstärkungsfasern. Hierbei empfehlen sich insbesondere Polysaccharid- und Proteinfasern, wie z.B. Kollagenfasern, Seide und Baumwollfasern, sowie Polylactidfasern oder auch Mischungen hiervon.

Andererseits sind molekulare Verstärkungsstoffe ebenso geeignet, um die mechanischen Eigenschaften und, falls gewünscht, auch die Resorptionsstabilität der Nervenleitschiene zu verbessern.

Bevorzugte molekulare Verstärkungsstoffe sind insbesondere Polylactidpolymere und deren Derivate, Cellulosederivate und Chitosan und dessen Derivate. Auch lassen sich die molekularen Verstärkungsstoffe als Mischungen einsetzen.

Bevorzugt wird der Formkörper der Nervenleitschiene mindestens einen Teil des oder der Verstärkungsstoffe umfassen. Die Verstärkungsstoffe sind dabei in einer Matrix aus dem Gelatine basierenden Material eingebettet oder liegen in einer molekularen Mischung mit dem Gelatine basierenden Material vor.

Bevorzugte Ausführungsformen der Nervenleitschienen weisen einen mehrlagigen Formkörper auf, wodurch es möglich ist, die einzelnen Lagen mit spezifischen Funktionen auszustatten, wie weiter unten noch näher erläutert werden wird. Beispielsweise kann eine der Lagen als die semipermeable Schicht fungieren.

Überraschenderweise hat sich herausgestellt, dass Gelatine basierendes Material, insbesondere hochmolekulare Gelatine, eine Angiogenese fördernde Wirkung aufweist, sodass gleichzeitig mit dem Implantieren der erfindungsgemäßen Nervenleitschiene neben der eigentlichen Basisfunktion der Bereitstellung einer Leitschiene für das Nervenwachstum auch die Ausbildung von Blutgefäßkapillaren gefördert wird, sodass die Umgebung der Nervenleitschiene, d.h. des sich neu entwickelnden Axons, verstärkt mit Nährstoffen versorgt wird.

Bei der erfindungsgemäßen Nervenleitschiene wird das Gelatine basierende Material Gelatine als Hauptkomponente umfassen, das bedeutet, dass gegenüber möglichen anderen weiteren Komponenten wie z.B. anderen resorbierbaren Biopolymeren wie z.B. Polysacchariden oder Hyaluronsäure Gelatine den größten Anteil an dem Material stellt. Bei dieser Bemessungsregel wie auch bei den im Folgenden noch gegebenen spezifischeren Empfehlungen bleiben eventuelle Anteile der Verstärkungsstoffe unberücksichtigt.

Weiter bevorzugt stellt die Gelatine den überwiegenden Anteil an dem Gelatine basierenden Material.

Noch weiter bevorzugt stellt die Gelatine im Wesentlichen das gesamte Gelatine basierende Material.

Die bevorzugt verwendete hochmolekulare Gelatine in dem Gelatine basierenden Material weist vorzugsweise einen Bloomwert im Bereich von ca. 160 g bis 300 g auf.

Versuche mit niedermolekularen Gelatineanteilen zeigen, dass deren angiogenetisch fördernder Effekt deutlich geringer ist als der der hochmolekularen Gelatine.

Auch bietet die hochmolekulare Gelatine in dem Gelatine basierenden Material weitere Vorteile, die im Folgenden bei der Frage der Einstellung des Vernetzungsgrades noch näher besprochen werden.

Weiter bevorzugt wird eine Gelatine verwendet, die endotoxinarm ist, wobei hier insbesondere Schweineschwartengelatine geeignet ist. Eine solche Gelatine weist bevorzugt einen Endotoxingehalt, gemessen nach dem LAL-Test (vgl. vierte Ausgabe des Europäischen Arzneibuches Ph. Eur. 4) von 1.200 I.E./g oder weniger auf, insbesondere auch 200 I.E./g oder weniger. Durch besonders sorgfältiges Arbeiten lassen sich sogar Endotoxingehalte von z.B. 140 I.E./g oder sogar bis zu 50 I.E./g erzielen.

Gelatine anderer Herkunft oder nach anderen Methoden hergestellt kann Endotoxihwerte bis über 20.000 I.E./g aufweisen.

Erfindungsgemäß trägt eine sorgfältige Auswahl unter den zugelassenen Rohmaterialien, insbesondere nur frisch isolierte und angelieferte Schweineschwarte und der Ausschluss der Verwendung von Kühlware, der sofortige Einsatz des Rohmaterials ohne längere Transport- oder Lagerdauern, eine gesonderte Reinigung der gesamten Prozessanlage vor Produktionsbeginn der Spezialchargen, gegebenenfalls einschließlich des Einsatzes von Ionentauschern und Filtersystemen, zu einer sehr drastischen Absenkung der Endotoxinwerte bei.

Die erfindungsgemäßen Nervenleitschienen weisen typischerweise Längen im Bereich von 0,5 bis 50 cm auf. Im Falle, dass die Nervenleitschiene ein einzelnes Hohlröhrchen als Formkörper aufweist, weist dieser einen äußeren Durchmesser von ca. 1 bis 30 mm auf. Die Wandstärke, je nachdem ob der Formkörper ein- oder mehrlagig ausgebildet ist, beträgt beispielsweise 0,02 bis 5 mm.

Weist die Nervenleitschiene mehrere Hohlröhrchen/Formkörper auf, so liegt der äußere Durchmesser jeweils bevorzugt im Bereich von 100 bis 800 µm. Im normalen Nerv liegen kleinere Gruppen von Axonen in so genannten Faszikeln vor. Eine Nervenleitschiene mit mehreren Formkörpern mit den vorstehend angegebenen bevorzugten Durchmessern bildet diese Struktur nach.

Die Herstellung von röhrchenförmigen Hohlkörpern aus Gelatine basierenden Materialien stellt eine besondere Herausforderung dar. Insbesondere die Herstellung von Nervenleitschienen für die Nervenregeneration im peripheren Nervensystem verlangt nach relativ kleinen Abmessungen. Gleichzeitig sollen die Abmessungen reproduzierbar erhalten werden und das Herstellungsverfahren nicht allzu aufwändig sein.

Ein bevorzugtes Verfahren stellt das Tauchverfahren dar, bei dem ein Dorn ein- oder mehrfach in eine Lösung des Gelatine basierenden Materials eingetaucht und dazwischen mindestens partiell trocknen gelassen wird.

Die Ablösung der so erzeugten Hohlröhrchen als Formkörper für erfindungsgemäße Nervenleitschienen gestaltet sich jedoch schwierig aufgrund der geringen Durchmesser und Wandstärken.

Bevorzugt wird deshalb ein Hohlkörper mit größerem Durchmesser und Wandstärke in einem ersten Schritt hergestellt und dieser Hohlkörper dann zu einem Hohlröhrchen mit dem gewünschten äußeren Durchmesser und der angestrebten Wandstärke in seiner Längsrichtung verstreckt.

Die Verstreckung von Gelatine basierenden Materialien in merklichem Umfang ist bisher in der Literatur nicht beschrieben. Es zeigt sich auch, dass ein Gelatine basierendes Material, insbesondere Gelatine selbst, ohne Modifikation nicht erfolgreich verstreckt werden kann.

Erfindungsgemäß wird das Gelatine basierende Material deshalb bevorzugt mit einem Anteil an Weichmacher eingesetzt, der im Bereich zwischen 12 und 40 Gew.%, insbesondere im Bereich von 16 bis 25 Gew.% liegt.

Gleichzeitig führt der Einsatz der Weichmacher erwartungsgemäß zu einer größeren Flexibilität der Nervenleitschiene, wodurch deren Handhabung beim Einsetzen als Implantat vereinfacht wird.

Erstaunlicherweise lässt sich Gelatine, die solche Anteile an Weichmachern enthält, in einem relativ großen Verstreckungsverhältnis verstrecken, welches in der Praxis von 1,4 bis 8 beträgt.

Die erfindungsgemäß bevorzugten Weichmacher für das Gelatine basierende Material werden insbesondere ausgewählt aus Glycerin, Oligoglycerinen, Oligoglycolen und Sorbit. Solche Weichmacher können in dem Gelatine basierenden Material verbleiben und werden im Körper des Patienten ebenso resorbiert wie das Gelatine basierende Material der Nervenleitschiene bzw. des Formkörpers selbst.

Erstaunlicherweise lässt sich durch den Einsatz der Weichmacher beim Verstrecken der Gelatine basierenden Materialien auch eine Zunahme der Reißfestigkeit in Längsrichtung der Nervenleitschiene erzielen und insbesondere auf einen Wert von 30 % oder mehr, insbesondere auch 50 % oder mehr steigern.

Dies bietet bei der Verwendung der erfindungsgemäßen Nervenleitschiene als Implantat bei der Nervenregeneration für den Chirurgen besondere Vorteile, da er ein relativ unempfindliches Implantat vorliegen hat. Ebenfalls lässt sich durch das Verstrecken des vernetzten Materials eine Reißfestigkeit in Längsrichtung von 40 N/mm² oder mehr, insbesondere auch 60 N/mm² oder mehr erzielen.

Vorzugsweise wird das Gelatine basierende Material in zumindest partiell vernetztem Zustand verwendet. Über den Vernetzungsgrad lässt sich die Resorptionsstabilität der Nervenleitschiene bzw. des Formkörpers einstellen.

Die Vernetzung wird bevorzugt die in dem Gelatine basierenden Material enthaltene Gelatine betreffen.

Bei dem empfohlenen Tauchverfahren zur Herstellung von Hohlkörpern wird das Gelatine basierende Material bereits in der Lösung vorvernetzt eingesetzt. Dies führt zu einem gleichmäßigen Vernetzungsgrad im gesamten Gelatine basierenden Material.

Weiter bevorzugt wird der fertige, in der Regel verstreckte Formkörper in einer zweiten Stufe weiter vernetzt um so die gewünschte Resorptionsstabilität zu gewährleisten. Diese zweistufige Vernetzung erlaubt höhere Vernetzungsgrade als die einstufige Vernetzung. Hier ergibt sich dann auch die Möglichkeit gradierte Vernetzungsgrade zu realisieren.

Beispielsweise kann der Vernetzungsgrad des Gelatine basierenden Materials in der Wandung des Formkörpers benachbart zu der Außenoberfläche höher sein als in Bereichen der Wandung benachbart zum Lumen.

Aufgrund der Wahl eines unterschiedlichen Vernetzungsgrades an der Außenoberfläche des Formkörpers einerseits und benachbart zum Lumen des Formkörpers andererseits wird ermöglicht, dass bei der Regeneration der Nervenfasern, d.h. bei der Entwicklung der Axone, zum einen zunächst ein geschütztes Volumen in Form des Lumens angeboten wird, welches im Zuge des Nervenwachstums aufgrund fortschreitender Resorption des Gelatine basierenden Materials sich vergrößert. Trotzdem bleibt die Schutzfunktion der Nervenleitschiene weiter erhalten, da die außen liegenden Bereiche der Wandung des Formkörpers langsamer resorbiert werden und damit längere Zeit Schutz gegen das Einwandern von Fibroblasten gewähren können.

Dies erlaubt den sich regenerierenden Axonen zunehmend dicker werdende Myelinschichten zu bilden, die der Isolation dienen, ohne dass dadurch ein Druck in der Nervenleitschiene aufgebaut wird, der die sich regenerierenden Axone schädigen könnte.

Die mindestens partielle Vernetzung in der Lösung ebenso wie der zweite Vernetzungsschritt können sowohl chemisch als auch enzymatisch erfolgen.

Bei der chemischen Vernetzung kommen als Vernetzungsmittel insbesondere Aldehyde, Dialdehyde, Isocyanate, Carbodiimide und Alkylhalogenide zum Einsatz.

Besonders bevorzugt ist die Vernetzung mit Formaldehyd, da in dem jeweiligen Vernetzungsschritt gleichzeitig eine Sterilisierung des Gelatine basierenden Materials bzw. des Formkörpers erreicht wird.

Bei der enzymatischen Vernetzung kommt vorzugsweise Transglutaminase zum Einsatz.

Für den Einsatzzweck der Nervenleitschiene wird der Vernetzungsgrad so gewählt, dass die Nervenleitschiene, insbesondere deren Formkörper, unter physiologischen Standardbedingungen (PBS-Puffer pH 7,2; 37°C) während 4 Wochen eine Abnahme des Trockengewichts von höchstens ca. 20 Gew.% zeigt.

Das Lumen des Formkörpers ist verglichen mit den Abmessungen der natürlich vorkommenden einzelnen Nervenfasern extrem groß. Die Axone sind lediglich ca. 1 µm dick, während das von der Nervenleitschiene bereitgestellte Lumen einen Durchmesser von bis zu 10 mm aufweisen kann. Selbst bei der Verwendung von mehreren Hohlröhrchen in einer Nervenleitschiene bietet deren jeweiliges Lumen eine lichte Weite, die die Dicke der Axone um ca. 2 Größenordnungen oder mehr übersteigt.

Um das zielgerichtete Wachstum der Axone während der Regeneration weiter zu fördern und die Regenerationszeit möglichst kurz zu halten, werden bevorzugt ein oder mehrere Leitelemente im Lumen des Formkörpers parallel zu dessen Längsrichtung angeordnet. Das oder die Leitelemente erstrecken sich dabei bevorzugt im Wesentlichen über die gesamte Länge des Lumens.

Die Leitelemente können dabei bereits mit Hilfszellen, insbesondere den Schwann-Zellen, besiedelt sein, die das Axonwachstum fördern.

Im natürlichen Nerv finden sich Schwann-Zellen, die über geregelte Rückkopplungsschleifen Wachstumsfaktoren abgeben und sowohl die Blutgefäßbildung wie auch die axonale Regeneration fördern. Diese Zellen können z.B. aus dem verletzten Nerv eines Patienten isoliert werden und über die erfindungsgemäße Nervenleitschiene nach *in vitro* Kultivierung reimplantiert werden. Um die Schwann-Zellen möglichst gleichmäßig in dem Lumen der Nervenleitschiene verteilt anzuordnen, werden die Schwann-Zellen vorzugsweise in einer Gelatinegel-Matrix angeordnet, die bei erhöhter Temperatur (z.B. 40°C) sich verflüssigt und bei Abkühlen auf Körpertemperatur wieder geliert und die bei höherer Temperatur beigemischten Zellen immobilisiert. Auf diese Weise können die Schwann-Zellen gleichmäßig verteilt auf die Leitelemente aufgetragen werden und bleiben nach Abkühlen auf Raumtemperatur in diesem Zustand erhalten bis das Implantat beim Patienten eingesetzt wird.

Um das Wachstum der Axone und die Differenzierung des Nervengewebes nicht zu behindern, sollten die Leitelemente bevorzugt höchstens ca. 30 Vol.% des Lumens einnehmen.

Als Leitelemente eignen sich insbesondere Mikrofilamente mit durchschnittlichen Dicken von ca. 10 bis 100 µm, je nach der im Lumen zur Verfügung stehenden lichten Weite.

Um mehrere Mikrofilamente in dem Lumen gleich verteilt anordnen zu können werden diese vorzugsweise in einer Matrix oder mittels Abstandhaltern auf Abstand gegeneinander stabilisiert.

Eine besonders ausgeprägte Führungsfunktion beobachtet man bei Leitelementen, die Führungsrillen aufweisen. Hieraus ergibt sich ein stereotropischer Effekt und es können schon die optional verwendeten Hilfszellen wie in den so genannten Büngnerschen Bändern mit einer sehr guten Längsausrichtung an-gesiedelt werden. In der Folge erhält man ein ebenfalls hochgradig längs orientiertes Wachstum der Axone. Die geometrischen Abmessungen sind nicht besonders kritisch und können z.B. eine Tiefe von 0,5 bis 50 µm aufweisen. Wichtiger ist das Vorhandensein von Kanten, die die Führungsrillen begrenzen.

Verwendet man eine Matrix um die Mikrofilamente gegeneinander zu fixieren, sollte das Matrixmaterial vorzugsweise aus einem Material gebildet sein, welches ein Axonwachstum hemmt, so dass deren Wachstum sich alleine an den Mikrofilamenten ausrichtet. Axonwachstum hemmende Materialien sind z.B. Hyaluronsäure oder auch hydrophobierte Gelatinegele.

Alternativ kann ein Leitelement aus einem aufgewickelten Flächenmaterial hergestellt sein, welches zwischen seinen Wickellagen Mikrokanäle bereitstellt, ähnlich den Führungsrillen der zuvor beschriebenen Mikrofilamente. Die notwendige Struktur kann dem Flächenmaterial beim Gießen desselben in einer Form oder durch nachträgliches Verprägen, Stanzen oder dergleichen mitgegeben werden. Die Wickelachse für das Flächenmaterial ist parallel zur Längsrichtung der Nervenleitschiene.

Bei entsprechender Ausgestaltung des Flächenmaterials kann dieses gleichzeitig den Formkörper der Nervenleitschiene bilden.

Wie bereits weiter oben angesprochen kann die Nervenleitschiene mehrere Formkörper enthalten, die vorzugsweise mittels eines Matrixmaterials miteinander verbunden werden. Dieses Matrixmaterial enthält vorzugsweise Angiogenese fördernde Komponenten, um das Einsprossen von Blutgefäßen zwischen den Axonen zu erlauben, ähnlich wie dies auch in den natürlichen Faszikeln in einer nichtneuronalen Matrix vorkommt. Dieses Matrixmaterial ist vorzugsweise Gelatine basierendes Material und weist besonders bevorzugt eine offenporige Struktur auf.

Dieses Matrixmaterial nimmt beispielsweise einen Volumenanteil von 30 bis 60 Vol.% des Volumens der Nervenleitschiene ein. Das Matrixmaterial ist weiter bevorzugt resorbierbar.

In einer weiteren bevorzugten Ausführungsform der Erfindung weist die Nervenleitschiene eine den oder die Formkörper umgebende Außenhülle oder Mantelmatrix auf, welche ebenfalls aus einem resorbierbaren Material gebildet ist. Insbesondere empfiehlt sich für die Mantelmatrix eine poröse, insbesondere offenporige Struktur. Die mittlere Porengröße liegt bevorzugt im Bereich von 100 bis 300 µm.

Die Mantelmatrix läßt sich beispielsweise durch Umschäumen des oder der Formkörper herstellen.

Alternativ kann die Mantelmatrix aus einem vorgefertigten festen Materialblock, insbesondere einem Schwamm, ausstanzen oder mittels eines Kern-Hülsenbohrers gewonnen werden. Die Durchgangsöffnung für das Einführen des Formkörpers, der gegebenenfalls zuvor mit der semipermeablen Schicht versehen wurde, kann einfach durch ein Bohrwerkzeug gebildet werden (beim Arbeiten mit dem Kern-Hülsenbohrer geschieht dies vorzugsweise gleichzeitig mit dem Erzeugen der Mantelmatrix).

In die so gebildete Mantelmatrix lässt sich der Formkörper einschieben. Ein leichter Klemmsitz zwischen Mantelmatrix und Formkörper reichen in aller Regel aus um die Nervenleitschiene sicher handzuhaben.

Vorzugsweise umfasst das Mantelmatrixmaterial eine Angiogenese fördernde Komponente um auch eine Blutgefäßbildung um die Nervenleitschiene herum und insbesondere bis an deren Formkörper heran zu fördern. Diese fördernde Wirkung wird vorzugsweise so realisiert, dass die Einsprossung von Blutgefäßen vor der Ausbildung oder zumindest während der Ausbildung der Axone im Lumen der Formkörper stattfinden kann. Geeignete Materialien hierfür sind wiederum Gelatine basierende Materialien, insbesondere auf der Basis von hochmolekularer Gelatine, wie sie weiter oben bereits ausführlich beschrieben wurde. Die Dicke der Außenhülle oder Mantelmatrix beträgt vorzugsweise 1 bis 2 mm.

Bei der Verwendung mehrerer Formkörper in der Nervenleitschiene kann die Mantelmatrix in die die Formkörper miteinander verbindende Matrix übergehen. Beide Matrices können aus demselben, insbesondere Angiogenese fördernden Material gebildet sein.

In diesem Fall ist dann die semipermeable Schicht Teil eines jeden Formkörpers um zu gewährleisten, dass in das dem Axonwachstum vorbehaltene Lumen kein Einwandern von Zellen stattfinden kann, die das Axonwachstum hemmen oder gar unterbinden.

Die Mantelmatrix kann dabei eine höhere Resorptionsrate aufweisen als der bzw. die Formkörper.

Je länger die von einem Implantat zu überbrückenden Strecken zwischen zwei Nervenstümpfen ist, desto länger sollte die Resorptionszeit der Gelatine basierenden Materialien der Formkörper sein. D.h. die Resorptionseigenschaft des Implantats, insbesondere der Formkörper und ganz besonders der semi-permeablen Schicht sollte abgestimmt sein auf die Länge der Nervendefektstrecke.

Bei Nervenleitschienen, die als Implantate für das periphere Nervensystem zum Einsatz kommen, ist die Resorptionseigenschaft der Nervenleitschiene so gewählt, dass die Resorption an einem Ende des Implantats beginnt und zum anderen Ende hin fortschreitet, entsprechend und vorzugsweise abgestimmt auf das Axonwachstum. Von dem Ende des Implantats, an dem die Axonentwicklung beginnt, beginnt auch die Myelinisierung und damit Verdickung der Nervenfaser. Durch die zeitlich abgestimmte Resorption des Implantats wird der notwendige Platz geschaffen, so dass eine Quetschung der Nervenfasern vermieden werden kann.

Bewerkstelligen kann man solche erwünschten Eigenschaften über den variablen Vernetzungsgrad des Gelatine basierenden Materials entlang der Längsrichtung der Nervenleitschiene, d.h. der Vernetzungsgrad ist an einem Ende der Nervenleitschiene geringer als am anderen Ende, wobei eine stufenförmige Variation oder im Wesentlichen kontinuierliche Zunahme des Vernetzungsgrades möglich ist.

Wird das Implantat dagegen im Zentralnervensystem eingesetzt, so wird dem Umstand Rechnung getragen, dass das Axonwachstum von beiden Nervenstümpfen aus erfolgt. Hier empfiehlt sich dann ein gradiertes Resorptionsverhalten, bei dem die Resorption an beiden Enden des Implantats ungefähr gleichzeitig beginnt und der mittlere Bereich zwischen den Enden des Implantats erst mit zeitlicher Verzögerung resorbiert wird.

Auch dies lässt sich durch eine entsprechende Gradierung des Vernetzungsgrades entlang der Längsrichtung der Nervenleitschiene bewerkstelligen. Auch hier kann eine stufenförmige Variation des Vernetzungsgrades gewählt werden oder eine im Wesentlichen kontinuierliche.

Aus praktischen Überlegungen kann es von Vorteil sein, wenn die lichte Weite des Lumen an den beiden Enden der Nervenleitschiene größer ist als im restlichen Bereich, wodurch sich die Nervenstümpfe der Läsion einfacher in die Nervenleitschiene einführen lassen.

Die Erfindung wird anhand der Zeichnung und der folgenden Beispiele noch näher erläutert. Es zeigen:
- Figur 1: eine schematische Darstellung einer erfindungsgemäßen Nervenleitschiene in einer ersten Ausführungsform;
- Figur 2: eine schematische Darstellung einer erfindungsgemäßen Nervenleitschiene in einer zweiten Ausführungsform;
- Figuren 3 und 4: Diagramme betreffend den Effekt erfindungsgemäß modifizierter Gelatine auf die Zellbesiedelung eines Substrats.
- Figur 5: eine schematische Darstellung eines Versuchsaufbaus zur Prüfung von Diffusionseigenschaften einer erfindungsgemäßen semipermeablen Schicht;
- Figuren 6 und 7: Diagramme mit Versuchsergebnissen der Prüfung der Diffusionseigenschaften einer erfindungsgemäßen semipermeablen Schicht;
- Figuren 8a und 8b: eine schematische Darstellung der Versuchsanordnung zur Untersuchung der Angiogenese mittels einer Choriollantois-Membran;
- Figur 9: ein Diagramm zur Veranschaulichung der Entwicklung von Blutgefäßen im Angiogenese fördernden Material; und
- Figur 10: lichtmikroskopische Aufnahmen von Schwann-Zellen und Axonen auf einer inerten Kunststofffolie kultiviert.

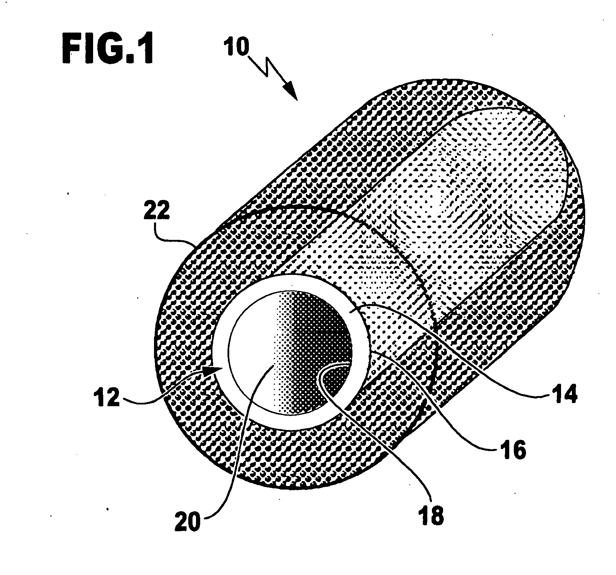

Figur 1 zeigt eine insgesamt mit dem Bezugszeichen 10 bezeichnete Nervenleitschiene mit einem Formkörper 12 aus einem vernetzten, Gelatine basierenden Material. Der Formkörper 12 weist einen röhrchenförmigen Hohlkörper mit einer Wandung 14 auf, welche eine Außenoberfläche 16 aufweist und mit einer Innenoberfläche 18 einen Lumen genannten Hohlraum 20 definiert.

Die Nervenleitschiene weist ferner eine semipermeable Schicht auf, welche (in der Figur 1 nicht im Einzelnen dargestellt) integral mit dem Formkörper 12 ausgebildet ist. Die Position semipermeablen Schicht liegt innerhalb der Wandung 14, vorzugsweise benachbart zur Außenoberfläche 16.

Die Außenoberfläche des Formkörpers 12 ist von einer Außenhülle 22 umgeben, welche offenporig ausgebildet ist und eine Angiogenese fördernde Komponente, insbesondere eine hochmolekulare vernetzte Gelatine mit Schwammstruktur, umfasst.
Die Außenhülle 22 kann aus einem Schwammmaterial aus vernetzter Gelatine hergestellt werden, wie dies im folgenden Beispiel 3 im Einzelnen beschreiben ist. Zunächst wird ein Block eines solchen Schwammmaterials in ausreichender Dicke hergestellt (entsprechend der Länge der fertigen Nervenleitschiene). Aus diesem Block wird z. B. durch Stanzen oder mittels eines Kern-Hülsenbohrers die Außenhülle 22 als Hohlzylinder gefertigt. In die Durchgangsöffnung dieses Hohlzylinders kann dann der Formkörper 12 eingeschoben werden, der in der Außenhülle 22 bevorzugt in einem leichten Klemmsitz gehalten wird.

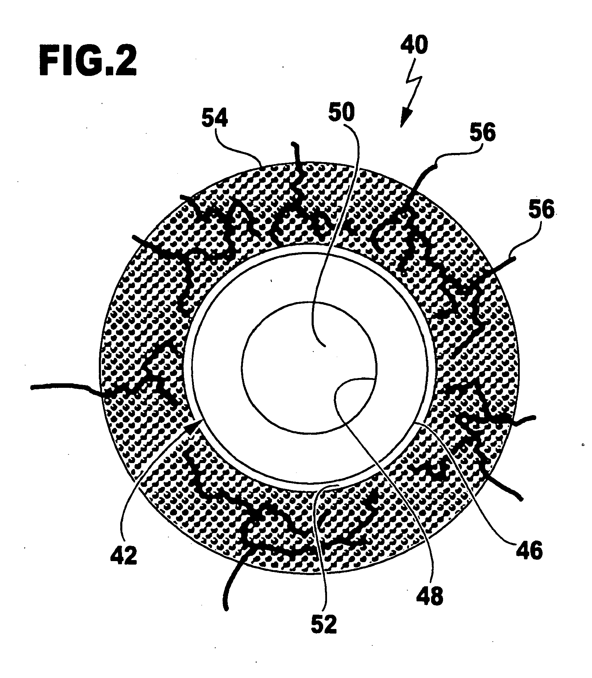

Figur 2 zeigt eine weitere erfindungsgemäße Nervenleitschiene 40 mit einem röhrchenförmigen Hohlkörper als Formkörper 42. Der Formkörper 42 weist eine Wandung 44 aus einem Gelatine basierenden Material auf mit einer Außenoberfläche 46 und einer ein Lumen 50 definierenden Innenoberfläche 48.

An die Außenoberfläche 46 des Formkörper 42 angrenzend ist eine separate semipermeable Schicht 52 angeordnet, welche Nährstoffe und Gase hindurch diffundieren lässt, jedoch das Eindringen von Zellen, insbesondere Fibroblasten blockiert.

Nach außen anschließend ist der Formkörper 42 mit der semipermeablen Schicht 52 von einer Außenhülle 54 umgeben, welche ähnlich wie in bei der Ausführungsform der Figur 1 ausgebildet ist. Die Außenhülle 54 kann wie im Zusammenhang mit der Ausführungsform von Figur 1 beschrieben gefertigt und auf den Formkörper 42 aufgeschoben werden.

Figur 2 zeigt schematisch das bereits erfolgte Einsprießen von Blutgefäßen 56 in die Außenhülle, gefördert durch die Angiogenese fördernde Komponente der Außenhülle 54.

### Beispiele

### Beispiel 1: Herstellung einer erfindungsgemäßen Nervenleitschiene

### Herstellung eines röhrchenförmigen Hohlkörpers

Im Folgenden wird zunächst die Herstellung eines röhrchenförmigen Hohlkörpers beschrieben, der Grundbestandteil einer erfindungsgemäßen Nervenleitschiene ist. Die verschiedenen hergestellten Typen weisen Innendurchmesser von etwa 2.000 µm, 1.100 µm und 150 µm und werden mittels des erfindungsgemäß bevorzugten Tauchverfahrens hergestellt und nachfolgend verstreckt.

Hierfür wurden zunächst 100 g Schweineschwartengelatine (Bloomstärke 300 g) in einer Mischung aus 260 g Wasser und 40 g Glycerin als Weichmacher bei 60 °C gelöst und die Lösung mittels Ultraschall entgast. Dies entspricht einem Anteil an Weichmacher in dem Material von ca. 29 Gew.%, bezogen auf die Masse von Gelatine und Glycerin.

Nach Zugabe von 4 g einer wässrigen, 2,0 Gew.%igen Formaldehydlösung (800 ppm Vernetzer bezogen auf die Gelatine) wurde die Lösung homogenisiert, nochmals entgast und die Oberfläche von Schaum befreit. Dann wurde ein Edelstahldorn als Formelement mit einem Durchmesser von 2 mm, der zuvor mit einem Trennwachs eingesprüht worden war, in einer Länge von ca. 3 cm kurz in die so hergestellte Lösung eingetaucht. Nach dem Herausziehen des Dorns aus der Lösung wurden dieser gedreht, so dass die anhaftende Lösung eine möglichst gleichmäßige Schicht bildete.

Nach etwa eintägigem Trocknen bei 25 °C und einer relativen Luftfeuchtigkeit von 30% konnte das gebildete Hohlröhrchen von dem Dorn abgezogen werden.

Das auf diese Weise hergestellte Hohlröhrchen hatte einen dem Dorndurchmesser entsprechenden Innendurchmesser von 2 mm und eine mittlere Wandstärke von 300 µm, welche lichtmikroskopisch ermittelt wurde.

Um das Hohlröhrchen auf einen kleineren Innendurchmesser zu bringen, wurde es fünf Tage bei 23°C und einer relativen Luftfeuchtigkeit von 45 % gelagert und anschließend verstreckt.

Zum Verstrecken wurde das Röhrchen an beiden Enden eingespannt und unter Einwirkung von heißem Wasserdampf erweicht. In diesem thermoplastischen Zustand wurde es mit einem Verstreckungsverhältnis von ca. 1,4 gelängt, in diesem Zustand fixiert und während 16 Stunden bei 23 °C und einer relativen Luftfeuchtigkeit von 45% getrocknet.

Das dabei erhaltene Hohlröhrchen wies einen Innendurchmesser von ca. 1.100 µm und eine Wandstärke von ca. 200 µm auf. Eine lichtmikroskopische prüfung ergibt eine äußerst regelmäßig ausgebildete Querschnittsform als auch eine sehr gleichförmige Wandstärke des Röhrchens über dessen Umfang und Länge gesehen.

Mit größeren Verstreckungsverhältnissen wurden Hohlröhrchen mit Innendurchmessern von bis zu 150 µm erhalten.

Um die physiologische Abbauzeit der Röhrchen zu verlängern, wurde die darin enthaltene Gelatine einem weiteren Vernetzungsschritt unterzogen. Hierzu wurden die Röhrchen in einem Exsikkator für 17 Stunden dem Gleichgewichtsdampfdruck einer 17 Gew.%igen wässrigen Formaldehydlösung bei Raumtemperatur ausgesetzt.

Dabei können die Enden der Röhrchen verschlossen werden, so dass die Vernetzung nur von der Außenoberfläche her erfolgt. Hier findet man dann einen höheren Vernetzungsgrad an der Außenoberfläche verglichen mit der das Lumen des Röhrchens definierenden Innenoberfläche und eine entsprechend erhöhte Resorptionsstabilität.

Ein Hohlröhrchen mit einem höheren Vernetzungsgrad im Wandbereich benachbart zu Lumen kann z.B. dadurch erhalten werden, dass der Formaldehyddampf ausschließlich durch das Lumen des Hohlröhrchens geleitet wird.

Alternativ oder ergänzend können unterschiedliche Vernetzungsgrade auch so realisiert werden, dass der Dorn nacheinander in Lösungen mit verschiedenen Konzentrationen an Vernetzungsmittel eingetaucht wird. Dadurch ergibt sich ein entsprechend gradierter Vernetzungsgrad über die Wanddicke des Hohlröhrchens.

Es versteht sich, dass die Eigenschaften des hier beschriebenen Hohlröhrchens in vielfältiger Weise modifiziert werden können, indem insbesondere die Größe und Gestalt des Dorns, die Anteile an Gelatine, Weichmacher und Vernetzungsmittel in der Lösung, die Anzahl der Tauchgänge, und die Intensität der Nachvernetzung an die jeweiligen Erfordernisse angepasst werden.

### Erzeugen der semipermeablen Schicht

Die Außenoberfläche der oben beschriebenen Hohlröhrchen kann z.B. chemisch modifiziert werden um eine das Lumen umgebende, semipermeable Schicht integral mit dem Hohlröhrchen zu erzeugen.

So lassen sich beispielsweise die Aminogruppen der Lysinreste mittels Bernsteinsäureanhydrid in eine succinierte Form überführen, wodurch der pKₛ-Wert des Gelatinematerials von 8 bis 9, wie er für die unmodifizierte Gelatine gefunden wird, auf ca. 4 abgesenkt wird.

Eine weitere Möglichkeit die Gelatine zu modifizieren besteht darin, die Aminogruppen der Lysinreste in Dodecenyl-succinyl-Gruppen zu überführen. Der pKₛ-Wert wird hierbei auf ca. 5 abgesenkt und gleichzeitig findet eine leichte Hydrophobierung der Gelatine statt.

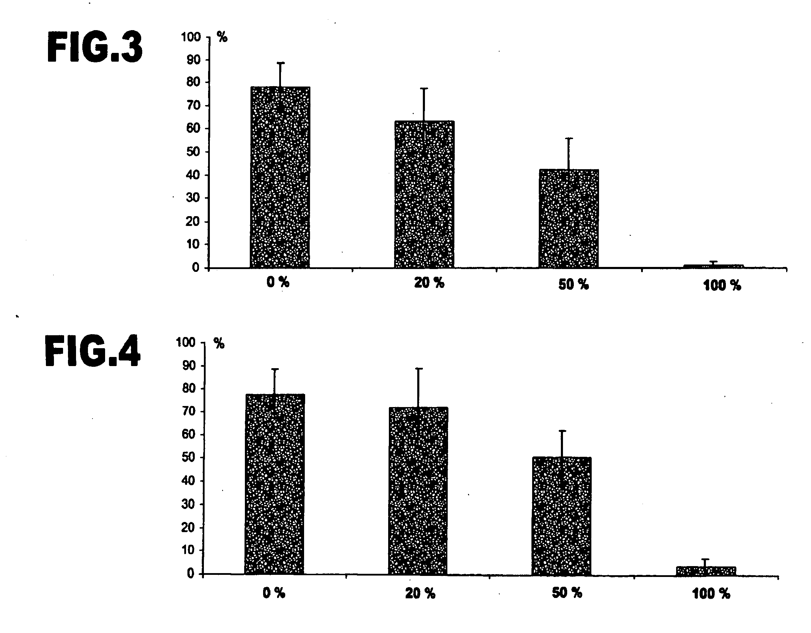

In beiden Fällen nimmt die Zelladhäsion von Fibroblasten gegenüber einer solchermaßen behandelten Außenoberfläche deutlich ab, was in den nachfolgend beschriebenen Versuchen und im Zusammenhang mit den Figuren 3 und 4 noch näher erläutert wird.

Zunächst sei der Vollständigkeit halber noch erwähnt, dass bei den wie oben beschrieben erzeugten Hohlröhrchen alternativ zu der Modifikation der Gelatine der Außenoberfläche diese mit einer gesonderten semipermeablen Schicht versehen werden kann. Um bei den zuvor gewählten Beispielen zu bleiben, kann diese Schicht durch das Auftragen einer succinierten oder Dodecenyl-succinierten Gelatine oder Abmischungen hiervon mit anderen Biopolymeren, insbesondere auch unmodifizierter Gelatine in wässriger Lösung erfolgen. Die Vorgehensweise kann hierbei an das Tauchverfahren angelehnt sein wie es weiter oben für die Herstellung der Hohlröhrchen beschrieben wurde.

Der Umsetzungsgrad der Lysingruppen der modifizierten Gelatine beträgt vorzugsweise 30 % oder mehr.

Im Falle der Dodecenyl-succinierten Gelatine reichen oft Umsetzungsgrade von 40 bis 50 % sehr gut aus, während bei der succinierten Gelatine eher eine 80 %ige bis nahezu vollständige Umsetzung der Lysingruppen beste Resultate liefert.

Die Figuren 3 und 4 zeigen Zelladhäsionsergebnisse für zu Testzwecken auf Glasoberflächen aufgebrachten Prüfflächen aus Gelatinematerialien, die ausgehend von Schweineschwartengelatine (MW 119 kDa) und einer zu ca. 95 % an den Lysingruppen succinierten Gelatine (Figur 3) bzw. ca. 45 % Dodecenyl-succinierten Gelatine (Figur 4) gleichen Typs hergestellt wurden. Es wurden jeweils Abmischungen von unmodifizierter Gelatine mit modifizierter Gelatine in den Verhältnissen 100:0, 80:20, 50:50 und 0:100 geprüft.

In den Tests wurden jeweils 20.000 porciner Chondrozyten auf einer Prüffläche während 4 h bei 37°C inkubiert. Der Überstand wurde entfernt, die Oberfläche gewaschen und die auf der Oberfläche verbleibenden Zellen fixiert um sie anschließend lichtmikroskopisch auszuwerten. Vergleichbare Ergebnisse wurden mit humanen Chondrozyten erhalten.

Die Prozentangaben in den Diagrammen stehen für den Anteil der auf den Folienprüfflächen gefunden Zellen gegenüber der zur Inkubation benutzten Anzahl, nachdem die vorstehend genannte Prozedur durchgeführt wurde.

Für beide modifizierte Gelatinearten ergaben sich Besiedelungseffekte nahe Null bei der ausschließlichen Verwendung von modifizierter Gelatine.

Hieraus läßt sich für den Fall der Modifikation der Oberflächen der Hohlröhrchen schließen, dass bei einem entsprechend hohen Umsetzungsgrad der an der Außenoberfläche zugänglichen Lysingruppen vergleichbare Effekte erzielbar sind.

Entsprechendes gilt natürlich für ein Aufbringen einer gesonderten Schicht aus modifizierter Gelatine auf die Außenoberfläche des Hohlröhrchens.

Da ein Einwandern von Zellen in die Wandung des Hohlröhrchens zunächst deren Adhäsion an der Außenoberfläche voraussetzt, sind die Bedingungen für eine Sperrwirkung für Zellen, wie sie erfindungsgemäß von einer semipermeablen Schicht erwartet werden, sehr gut erfüllt.

### Beispiel 2: Semipermeable Eigenschaft/Sperrschichtfunktion der Gelatinefolie anhand von Flächenmaterialtests

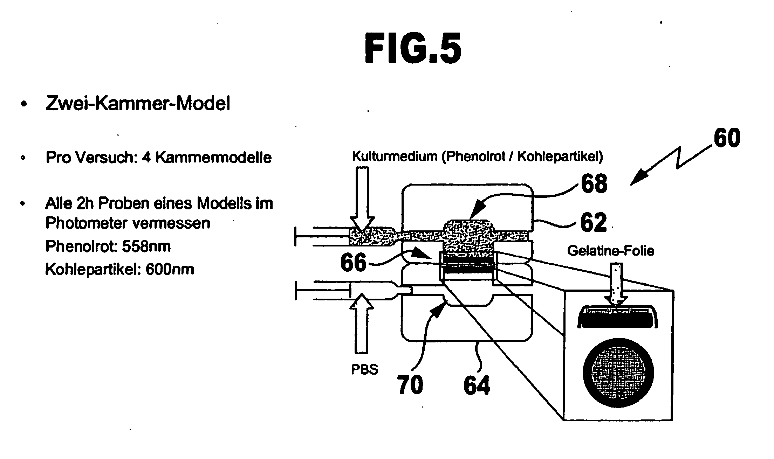

Zum Prüfen der Diffusionseigenschaften der oben beschriebenen Testfolien wurden diese in einer Zweikammer-Testvorrichtung 60 wie aus der Figur 5 ersichtlich zwischen zwei Blöcke 62 und 64 eingespannt, wobei beiderseits der Testfolie 66 Hohlräume 68, 70 in den Blöcken 62 bzw. 64 geschaffen wurden, die während der Versuchsphase mit unterschiedlichen Medien bespült wurden.

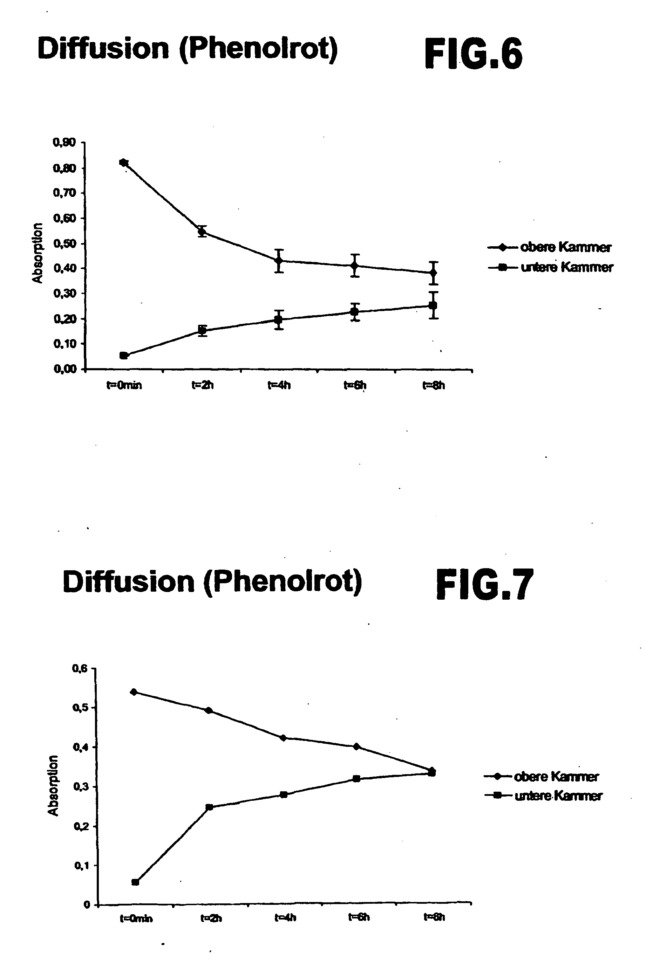

Die obere Kammer 68 wurde mit einer Phenolrotlösung als Nährlösungsersatz befüllt, in der unteren Kammer 70 wurde eine reine PBS-Lösung verwendet. Alle zwei Stunden wurde die Absorption der Phenolrot- und der PBS-Lösung gemessen. Die Messwerte sind in den Kurven der Figur 6 für eine Folie aus nicht modifizierter Gelatine wiedergegeben.

Der Versuch wurde mit einer Folie wiederholt, die zunächst mit 10.000 Zellen/cm² besiedelt wurde, die 2 h adhärieren gelassen wurden. Die adhärierten Zellen wurden in Kultur für eine Woche auf der Folie vermehrt, danach wurde dieselbe Messung wie oben beschrieben durchgeführt. Die Messwerte sind in der Figur 7 dargestellt.

Im Ergebnis findet man eine Abnahme der Phenolrotkonzentration in der oberen Kammer 68 und eine korrespondierende Zunahme der Phenolrotkonzentration in der unteren Kammer 70, entsprechend einer Nährstoffdiffusion durch die Folie. Im Falle der zellbesiedelten Folie ergibt sich in der Tendenz eine etwas beschleunigte Diffusion für das Phenolrot.

Parallel dazu wurde mit Kohlepartikelsuspensionen (Partikelgröße für 75 Gew.% kleiner 45 µm) keinerlei Durchgang durch die Folien gefunden. Dies bedeutet, dass auch die zellbesiedelte Folie noch eine wirksame Sperrschicht gegen das Eindringen von Zellen und Partikeln in der Größe von Zellen bietet und nicht durch zelluläre Proteasen außer Funktion gesetzt wird.

Die vorstehenden Ergebnisse ließen sich auch in zwei und drei Wochen dauernden Kulturversuchen bestätigen.

### Beispiel 3: Angiogeneseeffekt

### Herstellung und Eigenschaften von Formkörpern mit Zellstruktur auf Basis von vernetzter Gelatine

Es wurden fünf Ansätze einer 12 Gew.-%igen Lösung von Schweineschwartengelatine (Bloom-Stärke 300 g, mittleres Molekulargewicht 140 kDa) in Wasser durch Lösen der Gelatine bei 60 °C hergestellt, mittels Ultraschall entgast, und jeweils mit der entsprechenden Menge einer wässrigen Formaldehydlösung (1,0 Gew.-%ig, Raumtemperatur) versetzt, so dass 1500 ppm Formaldehyd (bezogen auf die Gelatine) vorlagen. Bei einem sechsten Ansatz erfolgte keine Zugabe von Formaldehyd.

Die homogenisierten Mischungen wurden auf 45 °C temperiert und nach einer Reaktionszeit von 10 min maschinell mit Luft aufgeschäumt. Der ca. 30minütige Aufschäumvorgang wurde für die sechs Ansätze mit einem unterschiedlichen Verhältnis von Luft zu Gelatinelösung durchgeführt, wodurch Zellstrukturen mit unterschiedlichen Nassdichten und Porengrößen gemäß Tabelle 1 erhalten wurden.

Die aufgeschäumten Gelatinelösungen, die eine Temperatur von 26,5 °C aufwiesen, wurden in Formen mit einer Abmessung von 40 x 20 x 6 cm gegossen und ca. vier Tage bei 26 °C und einer relativen Luftfeuchtigkeit von 10% getrocknet.

Die getrockneten Formkörper von allen sechs Ansätzen weisen eine schwammartige Zellstruktur auf (im Folgenden als Schwämme bezeichnet). Sie wurden in 2 mm dicke Schichten geschnitten und für den zweiten Vernetzungsschritt 17 Stunden in einem Exsikkator dem Gleichgewichtsdampfdruck einer 17 Gew.-%igen wässrigen Formaldehyd-Lösung bei Raumtemperatur ausgesetzt. Für den sechsten Ansatz stellte dies den ersten (und einzigen) Vernetzungsschritt dar. Um eine gleichmäßige Begasung des gesamten Volumens der Formkörper zu erreichen, wurde der Exsikkator dabei jeweils zwei- bis dreimal evakuiert und wieder belüftet.

Die Porenstruktur der Schwämme wurde lichtmikroskopisch ermittelt und konnte durch Rasterelektronenmikroskopie bestätigt werden.

**Tabelle 1**

| Ansatz | Nassdichte (mg/cm³) | Trockendichte (mg/cm³) | mittlere Porengröße (µm) |
|---|---|---|---|
| 1-1 | 100 | 20 | 250 |
| 1-2 | 175 | 27 | 200 |
| 1-3 | 300 | 50 | 125 |
| 1-4 | 530 | 70 | 100 |
| 1-5 | 600 | 100 | 75 |
| 1-6 | 78 | 12 | 300 |

Um die Stabilität der Schwämme zu bestimmen, wurden 30 x 30 x 2 mm große Stücke eingewogen, in je 75 ml PBS-Puffer gelegt und bei 37 °C gelagert. Nach der jeweiligen Lagerzeit wurden die Stücke 30 min in Wasser gewaschen, getrocknet und ausgewogen.

Während der Schwamm 1-6 nach drei Tagen bereits vollständig aufgelöst ist, sind alle zweistufig vernetzten Schwämme auch nach 14 Tagen noch zu über 80 % erhalten. Es zeigen sich jedoch erhebliche Unterschiede im weiteren Abbauverhalten, die auf die unterschiedlichen Aufschäumdichten der Materialien zurückzuführen sind. So ist der Schwamm 1-1 nach 21 Tagen und der Schwamm 1-2 nach 28 vollständig aufgelöst, während die Schwämme 1-4 und 1-5 auch nach 35 Tagen noch weitgehend erhalten sind. Daraus ergibt sich eine weitere Möglichkeit, das Abbauverhalten dieser Schwämme oder Zellstrukturmaterialien unabhängig von anderen Parametern gezielt zu beeinflussen.

Die Eigenschaften der Zellstrukturmaterialien können aber auch über eine Änderung der Gelatinekonzentration in der Ausgangslösung deutlich modifiziert werden.

Höhere Gelatinekonzentrationen führen zu breiteren (dickeren) Zellwänden oder Stegen zwischen den einzelnen Poren, was sich in einer erhöhten Bruchfestigkeit der entsprechenden Schwämme niederschlägt.

Über den Vernetzungsgrad, d.h. durch die Wahl der Konzentration des Vernetzungsmittels, kann hingegen die Stabilität der Formkörper, insbesondere im Hinblick auf proteolytischen Abbau, beeinflusst werden.

### Nachweis des Angiogenese fördernden Effekt

Aus analog der obigen Vorgehensweise erhältlichen, zweifach vernetzten Formkörpern (Trockendichte 22 mg/ml, mittlere Porengröße ca. 250 µm) wurden Proben hergestellt mit den Abmessungen 15 x 15 x 2 mm, im Folgenden Implantate genannt.

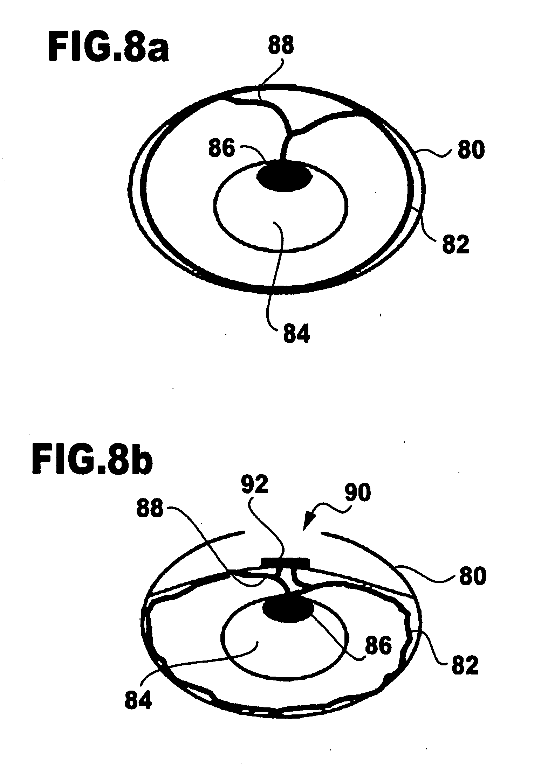

Die Angiogenese fördernden Eigenschaften dieser Implantate wurden mittels eines Versuches an befruchteten Hühnereiern, der schematisch in der Figuren 8a und 8b dargestellt ist, untersucht.

Figur 8a zeigt schematisch den Aufbau eines Hühnereis im Querschnitt. Unterhalb der Kalkschale 80 befindet sich die Choriallantois-Membran 82 (im Folgenden kurz CAM genannt). Ausgehend von dem am Rand des Eidotters 84 befindlichen Embryo 86 erfolgt eine Bildung von extraembryonalen Blutgefäßen 88, die sich entlang der CAM ausbreiten. Wird mittels einer Kanüle ein Teil des Eiweißes entnommen, kann anschließend ein Fenster 90 in die Kalkschale 80 geschnitten werden, ohne die CAM 82 zu verletzen (wie in Figur 8b dargestellt). Nun kann ein Implantat 92 auf die CAM 82 aufgelegt und dessen Wirkung auf die Blutgefäßbildung untersucht werden (vgl. z.B. J. Borges et al. (2004) Der Chirurg 75, 284-290).

Man beobachtet eine Umorientierung und Neubildung von Blutgefäßen in lichtmikroskopischen Aufnahmen nach 3, 5 und 7 Tagen.

Als Referenzbeispiele wurden neben dem erfindungsgemäßen Substrat vergleichbare schwammartige Materialien aus Kollagen (renaturiertes, bovines Kollagen, Dichte 5,6 mg/cm³, erhältlich von der Firma Innocoll) und Poly-DL-Lactid (Hersteller ITV Denkendorf) getestet.

Alle Implantate wurden auf eine CAM aufgelegt und nach 3, 4, 5, 6 und 7 Tagen die Anzahl der Blutgefäße bestimmt, die sich in direkter Umgebung der Implante entwickelt hatten. Es orientieren sich die Blutgefäße innerhalb weniger Tage sehr deutlich auf das Angiogenese fördernde Substrat bzw. die Referenzproben aus schwammartigem Kollagen und Poly-DL-Lactid hin.

Es zeigt sich, dass bei allen drei Proben gegenüber dem Nullwert (CAM ohne aufgelegtes Implant) eine merklich höhere Anzahl an Blutgefäßen vorhanden ist, wobei bei allen drei Proben ähnliche Effekte, insbesondere gesehen gegenüber dem Nullwert, erzielt wurden.

Das heißt, alle getesteten Materialien liegen in ihrer angiogenetischen Wirkung in ihrem Umfeld ungefähr auf dem gleichen erhöhten Niveau. Der beobachtete Effekt wird über eine gewisse Distanz hinweg verursacht und beruht deshalb vermutlich auf so genannten diffusiblen Faktoren.

Bei der CAM handelt es sich um ein Gewebe, das die Grenzfläche zwischen Luft und Eiflüssigkeit darstellt. Möglicherweise kommt es alleine durch den mechanischen Reiz durch das Auflegen des Substrates auf die CAM zu einer Aktivierung von Rezeptoren, was zu einer Ausschüttung von pro-angiogenetischen Faktoren wie z.B. VEGF der Zellen führen könnte. Hierdurch könnten Endothelzellen angelockt werden und es würde dann zu einer auf das Implantat gerichteten Blutgefäßbildung kommen.

Eine andere Erklärungsmöglichkeit besteht darin, dass aufgrund des Auflegens des Implantats der Zutritt von Luftsauerstoff zu dem Epithelgewebe behindert wird. So entsteht in der Region des Implantats eine so genannte Anoxie, da in dem Epithelgewebe weniger Sauerstoff zur Verfügung steht. Zellen reagieren auf eine Anoxie typischerweise mit der Ausschüttung von VEGF, wodurch eine Blutgefäßum- bzw. neubildung induziert wird. Dies bedeutet, dass der unterversorgte Teil der Zellen sich neue Versorgungsleitungen organisiert. Dieses biologische Phänomen tritt vermutlich oberhalb einer kritisch unterversorgten (deformierten) Gewebefläche auf.

Dies würde erklären, warum in Versuchen, bei denen beim bloßen Auflegen von schmalen Gummiringen auf die CAM (sehr kleine belegte Fläche) keine pro-angiogenetischen Effekte beobachtet werden konnten.

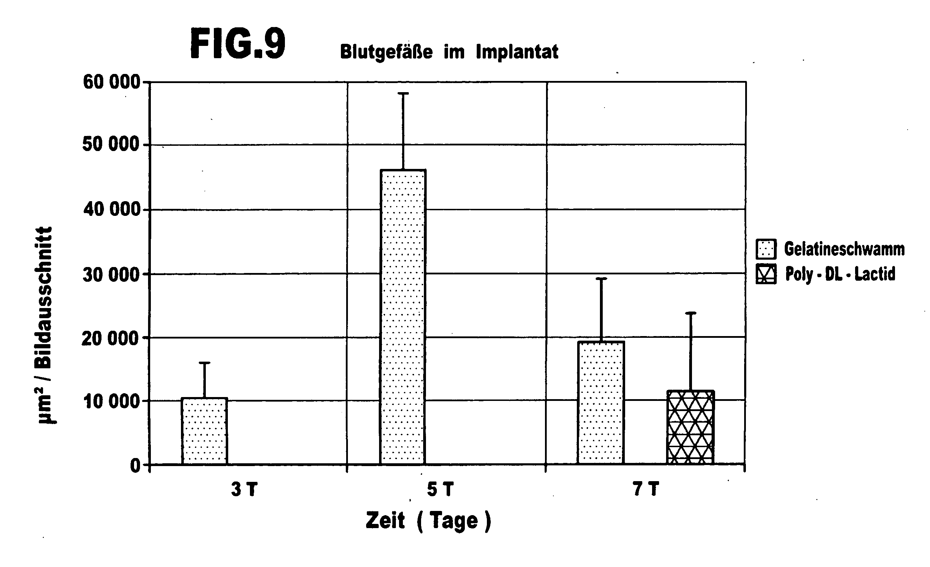

In der Figur 9 ist die Fläche der Blutgefäße (in µm²) innerhalb der Substrate oder Implantate der Vergleichsmaterialien und dem Angiogenese fördernden Substrat der vorliegenden Erfindung nach 3, 5 und 7 Tagen aufgetragen. In der Abfolge der dargestellten Säulen gilt die Reihenfolge Gelatineprobe, Kollagenprobe, Poly-DL-Lactidprobe.

Wie aus Figur 9 ersichtlich, zeigt sich nach 3 Tagen lediglich bei dem erfindungsgemäßen Angiogenese fördernden Substrat ein messbarer Anteil an Blutgefäßen im Implantat selbst, während in dem Kollagenschwamm und dem Poly-DL-Lactidschwamm keine messbaren Anteile an Blutgefäßen vorhanden sind.

Die messbaren Blutgefäße nach 5 Tagen zeigen eine extreme Steigerung bei den erfindungsgemäßen Angiogenese fördernden Substraten, während für die Poly-DL-Lactidprobe und für den Kollagenschwamm noch keinerlei Effekt beobachtet wird.

Nach 7 Tagen sinkt der Anteil an Blutgefäßen im Implantat bei dem erfindungsgemäßen Angiogenese fördernden Substrat deutlich ab, der Effekt ist aber immer noch etwa doppelt so hoch wie nach 3 Tagen. Zu diesem Zeitpunkt beobachtet man bei dem Kollagenschwamm immer noch keinen messbaren Erfolg, während sich bei dem Poly-DL-Lactidschwamm nun ein Effekt einstellt, wie er bei der erfindungsgemäßen Gelatineschwammimplantatprobe bereits nach 3 Tagen festzustellen war.

Zur Auswertung der Proben und Bestimmung der Anzahl der Blutgefäße im Implantat wurden von den jeweiligen Proben Gefrierschnitte angefertigt und mit DAPI gefärbt, um die Fläche der Blutgefäße innerhalb des Implantats zu analysieren. Dazu wurden Aufnahmen aus der mittleren Region der Schnitte gemacht und anschließend mit Bildverarbeitungsverfahren quantitativ ausgewertet. Bei Kollagenschwämmen konnte in der mittleren Region keinerlei Blutgefäßbildung beobachtet werden. Bei den Poly-DL-Lactidschwämmen war erst nach 7 Tagen, verbunden mit einer voranschreitenden Bindegewebszellenbesiedlung, Angiogenese festzustellen. Insgesamt schritt die Besiedlung mit Zellen aber auch bei dieser Vergleichsprobe deutlich langsamer voran als bei den erfindungsgemäßen Implantaten.

Die Rückbildung der Blutgefäße bei dem erfindungsgemäßen Implantat nach 7 Tagen drückt sich ein einer Verringerung der gemessenen Fläche aus. Dies könnte darauf beruhen, dass das Blutgefäßnetzwerk wieder soweit reduziert wird, wie es tatsächlich für die Implantbereiche benötigt wird, weil z.B. noch relativ wenige andere Zelltypen eingewandert sind, die versorgt werden müssen. Dies entspricht einem Vorgang, den man auch bei Infektionen findet, wo sich ein Blutgefäßnetzwerk wieder zurückbildet, sobald die Entzündung zurückgeht.

### Beispiel 4: Semipermeable Eigenschaft einer Gelatineröhre erlaubt Überleben eingekapselter Zellen

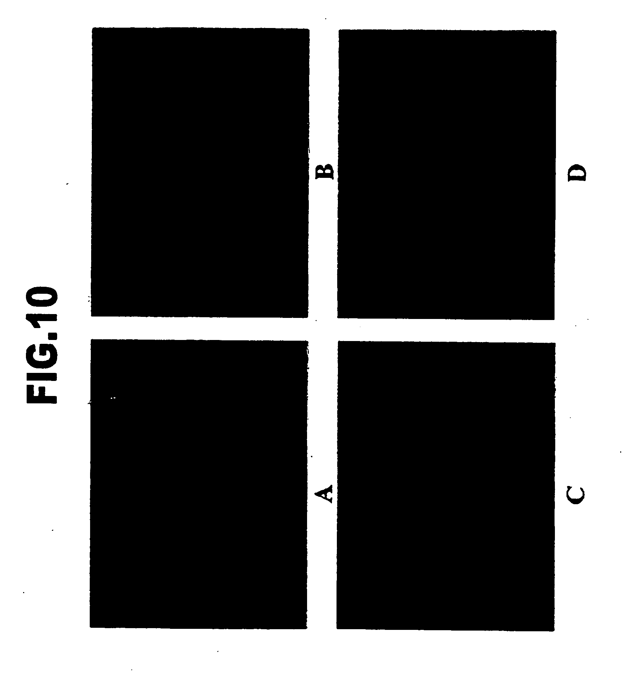

Es sollte untersucht werden, ob Zellen in einem verschlossenen röhrenförmigen Formkörper aus Gelatine (hergestellt gemäß Beispiel 1, 1100 µm Innendurchmesser, Wandstärke 200 µm, zweifach vernetzt) überleben. Dazu wurde der Formkörper eine Woche in PBS gelagert um ihn zu waschen. Anschließend wurden Schwann-Zellen auf 0,9 mm breite, transparente inerte Kunststoffstreifen (unbeschichtete Kopierfolie der Firma folex imaging X-70 mit einer Dicke von 0,1 mm) ausgesät. Der Kunststoffstreifen wurde zuvor mit dem PlasmaCleaner gereinigt, mit Poly-Lysin und Laminin (33µg/ml, 1h bei 37°C) beschichtet. Auf die Folie wurden zunächst aus dem Ischias-Nerv der Ratte isolierte Schwann-Zellen (25.000 Zellen/cm²) ausgesät und für 24h in Kultur gehalten. Danach wurden Einzelneurone aus Hinterwurzelganglien des peripheren Nervensystems präpariert und mit einer Dichte von 10.000 Zellen/cm² auf den Kunststoffstreifen mit die Schwann-Zellen ausgesät. Die Neurone konnten 4h adhärieren, im Anschluß wurde der Kunststoffstreifen mit den Zellen darauf in den Formkörper aus Gelatine eingeführt. Die Zellen nicht direkt, sondern auf dem transparenten Kunststoffstreifen in den Formkörper einzuführen, hatte den großen Vorteil, dass man den Kunststoffstreifen später wieder problemlos entnehmen konnte, um mikroskopisch die Zellvitalität zu bestimmen. Der röhrenförmige Formkörper wurde dann mit Stopfen aus Dentalwachs (Rosa Dura, Kem-Dent, GB) an den Enden verschlossen und für 5 Tage in Kultur genommen. Unter diesen Bedingungen konnten Nährstoffe und Sauerstoff nur noch durch die Wandung des Formkörpers zu den Zellen gelangen. Nach 5 Tagen Kulturzeit wurden die Kunststoffstreifen aus der Röhre entnommen und mit dem Antikörper SMI31 (Sternberger Monoclonals, USA) zum Nachweis von Axonen und DAPI zum Nachweis von Zellenkernen markiert. Als Kontrolle dienten Zell-besiedelte Kunststoffstreifen, die exakt gleich behandelt wurden, aber nicht verkapselt sondern offen 5 Tage im gleichen Medium (DMEM, 10% FCS, Glutamin, Gentamycin) kultiviert wurden. Wie auf den lichtmikros-kopischen Abbildungen der Figur 10 zu erkennen, überleben die Zellen/Neurone innerhalb und außerhalb des Formkörpers gleich gut (Abb. A und C). Dies ist an den hellen Punkten zu erkennen. Ebenso bilden sich in beiden Fällen ähnlich gut Axone aus (helle Fasern in Abb. B und D). Die Abbildungen A und B stellen Schwann-Zellen und Hinterwurzelganglienaxone der gleichen Kultur dar, die verkapselt war. Die Abbildungen C und D betreffen die nicht-verkapselte Kultur. Damit zeigte sich, dass die Permeabilität der Gelatineröhre ausreicht, um ein Überleben eingekapselter Zellen zu erlauben.

## Patentansprüche

1. Nervenleitschiene, umfassend einen Formkörper aus einem vernetzten, resorbierbaren, Gelatine basierenden Material, wobei der Formkörper ein röhrchenförmiger Hohlkörper ist mit einer Wandung mit einer Außenoberfläche und einer Innenoberfläche die ein Lumen definiert, wobei die Nervenleitschiene eine das Lumen umgebende, semipermeable Schicht umfasst, und wobei
(i) der Vernetzungsgrad der Nervenleitschiene bzw. deren Teile, insbesondere deren Formkörper, an einem Ende der Nervenleitschiene höher ist als am anderen Ende und in Richtung zu diesem anderen Ende hin in mehreren Stufen oder im wesentlichen kontinuierlich abnimmt; oder
(ii) der Vernetzungsgrad der Nervenleitschiene bzw. deren Teile, insbesondere deren Formkörper, an deren Enden geringer ist als im Bereich zwischen den Enden.

2. Nervenleitschiene nach Anspruch 1, **dadurch gekennzeichnet, dass** die semipermeable Schicht eine Gelstruktur als Zellbarriere umfaßt.

3. Nervenleitschiene nach Anspruch 1, **dadurch gekennzeichnet, dass** die semipermeable Schicht der Nervenleitschiene Poren aufweist, die im Mittel kleiner als 0,5 µm sind.

4. Nervenleitschiene nach Anspruch 1, **dadurch gekennzeichnet, dass** die semipermeable Schicht als Sperrschicht für positiv geladene Spezies, insbesondere Zellen, im Wesentlichen undurchlässig ausgebildet ist.

5. Nervenleitschiene nach Anspruch 1, **dadurch gekennzeichnet, dass** die semipermeable Schicht eine extrem hohe Hydrophilie aufweist.

6. Nervenleitschiene nach Anspruch 1, **dadurch gekennzeichnet, dass** die semipermeable Schicht leicht hydrophob ausgebildet ist.

7. Nervenleitschiene nach Anspruch 6, **dadurch gekennzeichnet, dass** die leicht hydrophobe, semipermeable Schicht mit Fettsäureresten modifizierte Gelatine umfasst.

8. Nervenleitschiene nach einem der Ansprüche 1 bis 7, **dadurch gekennzeichnet, dass** die Nervenleitschiene oder deren Formkörper an der Außenoberfläche immobilisierte Repulsionsproteine aufweist.

9. Nervenleitschiene nach einem der Ansprüche 1 bis 8, **dadurch gekennzeichnet, dass** die Nervenleitschiene einen Verstärkungsstoff umfasst.

10. Nervenleitschiene nach Anspruch 9, **dadurch gekennzeichnet, dass** der Verstärkungsstoff ausgewählt ist aus partikulären und/oder molekularen Verstärkungsstoffen.

11. Nervenleitschiene nach einem der Ansprüche 1 bis 10, **dadurch gekennzeichnet, dass** der Formkörper mehrlagig ausgebildet ist.

12. Nervenleitschiene nach Anspruch 11, **dadurch gekennzeichnet, dass** der Formkörper die semipermeable Schicht umfasst.

13. Nervenleitschiene nach einem der Ansprüche 1 bis 12, **dadurch gekennzeichnet, dass** der Formkörper in Richtung seiner Längsachse verstreckt ist.

14. Nervenleitschiene nach Anspruch 13, **dadurch gekennzeichnet, dass** das Verstreckungsverhältnis 1,4 bis 8 beträgt.

15. Nervenleitschiene nach einem der Ansprüche 1 bis 14, **dadurch gekennzeichnet, dass** das Lumen des Formkörpers ein oder mehrere in Längsrichtung des Formkörper ausgerichtete Leitelemente enthält.

16. Nervenleitschiene nach Anspruch 15, **dadurch gekennzeichnet, dass** die Leitelemente mit Hilfszellen, insbesondere Schwann-Zellen besiedelt sind.

17. Nervenleitschiene nach Anspruch 15 oder 16, **dadurch gekennzeichnet, dass** die Leitelemente Mikrofilamente umfassen.

18. Nervenleitschiene nach Anspruch 17, **dadurch gekennzeichnet, dass** die Mikrofilamente an ihrer Außenoberfläche Längsrillen aufweisen.

19. Nervenleitschiene nach einem der Ansprüche 16 bis 18, **dadurch gekennzeichnet, dass** ein Leitelement in Form eines Flächenmaterialwickels mit einer WIckelachse parallel zur Längsrichtung des Formkörpers in dem Lumen angeordnet ist, wobei in dem Wickel parallel zur Wickelachse eine Mehrzahl von Mikrokanälen ausgebildet ist.

20. Nervenleitschiene nach einem der Ansprüche 1 bis 19, **dadurch gekennzeichnet, dass** die Nervenleitschiene mehrere Formkörper in paralleler Anordnung umfasst.

21. Nervenleitschiene nach Anspruch 20, **dadurch gekennzeichnet, dass** die mehreren Formkörper mittels eines resorbierbaren Matrixmaterials miteinander verbunden sind.

22. Nervenleitschiene nach einem der Ansprüche 1 bis 21, **dadurch gekennzeichnet, dass** die Nervenleitschiene eine den oder die Formkörper umgebende resorbierbare Außenhülle, insbesondere mit einer porösen Struktur umfaßt.

23. Nervenleitschiene nach einem der Ansprüche 1 bis 22, **dadurch gekennzeichnet, dass** sie zur Überbrückung von Nervendefektstrecken, insbesondere nach Prostatektomien, Facialls Nervläsionen als Folge von Zahnextraktionen und Rückenmarksläsionen verwendbar ist.

24. Nervenleitschiene nach einem der Ansprüche 1 bis 22, **dadurch gekennzeichnet, dass** sie zur Induktion neuer Nervenbahnen verwendbar ist, insbesondere zur Umleitung von Nerven in Muskeln hinein um eine schmerzhafte Neurombildung zu vermeiden.

## Claims

1. A nerve guide comprising a shaped body made from a cross-linked, resorbable, gelatin-based material, the shaped body being a hollow body in the form of a tubule and having a wall with an external surface and an internal surface which defines a lumen, and the nerve guide comprising a semipermeable layer surrounding the lumen, wherein
(i) the degree of cross-linking of the nerve guide or its component parts, in particular its shaped body, is higher at one end of the nerve guide than that at the other end and reduces in a plurality of steps or continuously in the direction of the other end; or
(ii) the degree of cross-linking of the nerve guide or its component parts, in particular its shaped body, is less at its ends than it is in the region between the ends.

2. The nerve guide according to Claim 1, **characterized in that** the semipermeable layer has a gel structure as cell barrier.

3. The nerve guide according to Claim 1, **characterized in that** the semipermeable layer of the nerve guide has pores which are on average less than 0.5 µm.

4. The nerve guide according to Claim 1, **characterized in that** the semipermeable layer is formed to be substantially impermeable, as a barrier layer for positively charged species, in particular cells.

5. The nerve guide according to Claim 1, **characterized in that** the semipermeable layer is extremely hydrophilic.

6. The nerve guide according to Claim 1, **characterized in that** the semipermeable layer is formed to be slightly hydrophobic.

7. The nerve guide according to Claim 6, **characterized in that** the slightly hydrophobic, semipermeable layer comprises gelatin modified with esters of fatty acids.

8. The nerve guide according to any of Claims 1 to 7, **characterized in that** the nerve guide or its shaped body has immobilised repulsion proteins on the external surface.

9. The nerve guide according to any of Claims 1 to 8, **characterized in that** the nerve guide comprises a reinforcing material.

10. The nerve guide according to Claim 9, **characterized in that** the reinforcing material is selected from particulate and/or molecular reinforcing materials.

11. The nerve guide according to any of Claims 1 to 10, **characterized in that** the shaped body is formed with a plurality of layers.

12. The nerve guide according to Claim 11, **characterized in that** the shaped body comprises the semipermeable layer.

13. The nerve guide according to any of Claims 1 to 12, **characterized in that** the shaped body is stretched in the direction of its longitudinal axis.

14. The nerve guide according to Claim 13, **characterized in that** the stretch ratio is 1.4 to 8.

15. The nerve guide according to any of Claims 1 to 14, **characterized in that** the lumen of the shaped body has one or more guide elements aligned in the longitudinal direction of the shaped body.

16. The nerve guide according to Claim 15, **characterized in that** the guide elements are populated with auxiliary cells, in particular Schwann cells.

17. The nerve guide according to Claim 15 or 16, **characterized in that** the guide elements comprise microfilaments.

18. The nerve guide according to Claim 17, **characterized in that** the microfilaments have longitudinal grooves on their external surface.

19. The nerve guide according to any of Claims 16 to 18, **characterized in that** a guide element in the form of a roll of planar material which has an axis of rolling extending parallel to the longitudinal direction of the shaped body is disposed in the lumen, a plurality of micro-channels being formed in the roll, parallel to the axis of rolling.

20. The nerve guide according to any of Claims 1 to 19, **characterized in that** the nerve guide comprises a plurality of shaped bodies in parallel disposition.

21. The nerve guide according to Claim 20, **characterized in that** the plurality of shaped bodies are bonded to one another by means of a resorbable matrix material.

22. The nerve guide according to any of Claims 1 to 21, **characterized in that** the nerve guide comprises a resorbable outer sleeve surrounding the shaped body, the sleeve having in particular a porous structure.

23. The nerve guide according to any of Claims 1 to 22, **characterized in that** it is usable for bridging sections of nerves suffering from defects, in particular after prostatectomy, lesions of facial nerves as a result of tooth extraction and lesions of the spinal cord.

24. Nerve guide according to any of Claims 1 to 22, **characterized in that** it is usable for inducing new nerve pathways, in particular for diverting nerves into muscles in order to avoid formation of painful neuromas.

## Revendications

1. Canal de guidage pour les nerfs, comprenant un corps moulé constitué d'un matériau réticulé, résorbable et à base de gélatines, le corps moulé étant un corps creux tubulaire avec une paroi ayant une surface externe et une surface interne définissant un lumen, le canal de guidage pour les nerfs comprenant une couche semi-perméable entourant le lumen, et
(i) le degré de réticulation du canal de guidage pour les nerfs ou de ses parties, en particulier de son corps moulé, étant supérieur au niveau d'une extrémité du canal de guidage pour les nerfs à l'autre extrémité et diminuant dans la direction de cette autre extrémité par paliers ou sensiblement en continu ; ou
(ii) le degré de réticulation du canal de guidage pour les nerfs ou de ses parties, en particulier de son corps moulé, étant inférieur au niveau de ses extrémités à la zone située entre les deux extrémités.

2. Canal de guidage pour les nerfs selon la revendication 1, **caractérisé en ce que** la couche semi-perméable comprend une structure gélifiée à titre de barrière cellulaire.

3. Canal de guidage pour les nerfs selon la revendication 1, **caractérisé en ce que** la couche semi-perméable du canal de guidage pour les nerfs présente des pores qui sont en moyenne inférieurs à 0,5 µm.

4. Canal de guidage pour les nerfs selon la revendication 1, **caractérisé en ce que** la couche semi-perméable est conçue sous forme de couche de blocage, de manière à être essentiellement imperméable pour les espèces chargées positivement, en particulier les cellules.

5. Canal de guidage pour les nerfs selon la revendication 1, **caractérisé en ce que** la couche semi-perméable présente une hydrophilie extrêmement élevée.

6. Canal de guidage pour les nerfs selon la revendication 1, **caractérisé en ce que** la couche semi-perméable est conçue de manière à être légèrement hydrophobe.

7. Canal de guidage pour les nerfs selon la revendication 6, **caractérisé en ce que** la couche semi-perméable légèrement hydrophobe comprend des gélatines modifiées par des esters d'acides gras.

8. Canal de guidage pour les nerfs selon l'une des revendications 1 à 7, **caractérisé en ce que** le canal de guidage pour les nerfs ou son corps moulé présente des protéines de répulsion immobilisées sur la surface externe.

9. Canal de guidage pour les nerfs selon l'une des revendications 1 à 8, **caractérisé en ce que** le canal de guidage pour les nerfs comprend un matériau renforçateur.

10. Canal de guidage pour les nerfs selon la revendication 9, **caractérisé en ce que** le matériau renforçateur est choisi parmi les matériaux renforçateurs particulaires et/ou moléculaires.

11. Canal de guidage pour les nerfs selon l'une des revendications 1 à 10, **caractérisé en ce que** le corps moulé est conçu de manière à être multicouche.

12. Canal de guidage pour les nerfs selon la revendication 11, **caractérisé en ce que** le corps moulé comprend la couche semi-perméable.

13. Canal de guidage pour les nerfs selon l'une des revendications 1 à 12, **caractérisé en ce que** le corps moulé est allongé dans la direction de son axe longitudinal.

14. Canal de guidage pour les nerfs selon la revendication 13, **caractérisé en ce que** le rapport d'allongement va de 1,4 à 8.

15. Canal de guidage pour les nerfs selon l'une des revendications 1 à 14, **caractérisé en ce que** le lumen du corps moulé contient un ou plusieurs éléments de guidage orientés dans la direction longitudinale du corps moulé.

16. Canal de guidage pour les nerfs selon la revendication 15, **caractérisé en ce que** les éléments de guidage sont colonisés par des cellules de soutien, notamment des cellules de Schwann.

17. Canal de guidage pour les nerfs selon la revendication 15 ou 16, **caractérisé en ce que** les éléments de guidage comprennent des microfilaments.

18. Canal de guidage pour les nerfs selon la revendication 17, **caractérisé en ce que** les microfilaments présentent des sillons longitudinaux sur leur surface externe.

19. Canal de guidage pour les nerfs selon l'une des revendications 16 à 18, **caractérisé en ce qu'**un élément de guidage ayant la forme d'une bobine de matière plate est disposé dans le lumen avec un axe d'enroulement parallèle à la direction longitudinale du corps moulé, une majorité de microcanaux étant formée dans la bobine parallèlement à l'axe d'enroulement.

20. Canal de guidage pour les nerfs selon l'une des revendications 1 à 19, **caractérisé en ce que** le canal de guidage pour les nerfs comprend plusieurs corps moulés en disposition parallèle.

21. Canal de guidage pour les nerfs selon la revendication 20, **caractérisé en ce que** les multiples corps moulés sont reliés les uns aux autres au moyen d'un matériau de matrice résorbable.

22. Canal de guidage pour les nerfs selon l'une des revendications 1 à 21, **caractérisé en ce que** le canal de guidage pour les nerfs comprend une enveloppe extérieure résorbable, entourant le ou les corps moulés, notamment avec une structure poreuse.

23. Canal de guidage pour les nerfs selon l'une des revendications 1 à 22, **caractérisé en ce qu'**il est utilisable pour ponter les distances défectueuses des nerfs, en particulier après des prostatectomies, des lésions des nerfs de la face à la suite d'extractions dentaires et des lésions de la moelle épinière.

24. Canal de guidage pour les nerfs selon l'une des revendications 1 à 22, **caractérisé en ce qu'**il est utilisable pour l'induction de nouvelles voies nerveuses, notamment pour dévier des nerfs dans les muscles afin d'éviter la formation douloureuse de neuromes.
